# EUROPEAN PATENT SPECIFICATION

(11) **EP 0 401 262 B1**
(45) Date of publication and mention of the grant of the patent: **03.01.1996**
(21) Application number: 89902728.8
(22) Date of filing: 16.02.1989
(51) Int. Cl.: C07J 9/00

(54) **24R-SCYMNOL, AND PREPARATION AND USE THEREOF**
24R-SCYMNOL, HERSTELLUNG UND VERWENDUNG
COMPOSE DIT 24R-SCYMNOL ET PREPARATION ET UTILISATION DE CE COMPOSE

(30) Priority: 19.02.1988 AU 6850/88
(43) Date of publication of application: 12.12.1990
(73) Proprietor: J. W. BROADBENT NOMINEES PTY. LTD., AU-Melbourne, Victoria (AU)
(72) Inventor: KOSUGE, Yoshiki, Shizuoka 420 (JP); KOSUGE, Takuo, Shizuoka 420 (JP); TSUJI, Kuniro, Shizuoka 420 (JP); ISHIDA, Hitoshi, Shizuoka 420 (JP)
(74) Representative: Rickard, Timothy Mark Adrian
(86) International application number: AU8900064
(87) International publication number: WO8907607

(56) References cited:
- US-A- 3 931 403
- CHEMICAL ABSTRACTS, vol. 73, no. 11, 14th September 1970, page page 14, abstract no. 52330j, Columbus, Ohio, US; G.A.D. HASLEWOOD: "Bile alcohols", & BILE SALT METAB. 1969, 151-9
- ADVANCES IN BILE ACID RESEARCH., BILE ACID MEETING 3RD, 1974, pages 105-107; E.S. HASLEWOOD et al.: "Specificity and some characteristics of a 7alpha-hydroxysteroid dehydrogenase from E. coli"

## Description

This invention relates to the compound 24R-scymnol, to the preparation of this compound in substantially pure form, and to the use thereof, for example in the treatment of liver dysfunction.

In prior International Patent Application No. PCT/AU87/00281 there is disclosed a process for the isolation and preparation of an active principle by extraction from particular tissues of sharks. This active principle, now termed "isolutrol", was isolated in good yield from an aqueous extract of the livers and/or gall bladders of sharks, and the active component therein identified as 24R-(+)-3α,7α,12α,24,26- pentahydroxycoprostane-27-sodium sulphate ester (sodium 24R-scymnol sulphate). It has now been found that 24R-scymnol can be prepared from the active component disclosed in the prior International Patent Application, and that 24R-scymnol has activity in the treatment of liver dysfunction.

According to a first aspect of the present invention, there is provided the compound of the general formula I, in substantially pure form:

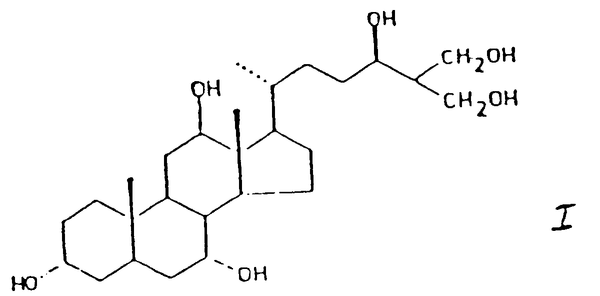

This compound, (24R)-(+)-5β-cholestane-3α,7α,12α, 24,26,27-hexol, has been designated as 24R-scymnol.

The chemical structure of scymnol has been reported by Bridgwater et.al. (Biochem.J. (1962) 82 : 285) as 5β-cholestane-3α,7α,12α,24ε,26,27-hexol. However, the stereochemical configuration at the 24-position of scymnol was not identified, and there are three possibilities in the configuration at this position, namely 24R, 24S or a mixture of 24R and 24S. Bridgwater et.al. also reported that chemically synthesized scymnol was exactly identical with scymnol derived from natural shark's bile. This suggests that scymnol is the mixture of 24R and 24S compounds, since theoretically the synthesized scymnol should be produced in both 24R and 24S configurations on reduction with NaBH₄ of the ketone in the 24-position of the starting material as described by Bridgwater et.al.

The present invention provides a method for the preparation of the compound of general formula I, which comprises the step of hydrolysis of a 24R-scymnol sulphate ester with an inorganic acid, preferably in the presence of barium chloride.

The process for the preparation of 24R-scymnol as broadly outlined above is particularly advantageous as it can be carried out using aqueous acids. In accordance with a preferred procedure of the present invention, 24R-scymnol has been prepared by hydrolysis of sodium scymnol sulphate with dilute hydrochloric acid in the presence of barium chloride, to give a crystalline product. The physical and chemical data of the product are set out in Table 1:

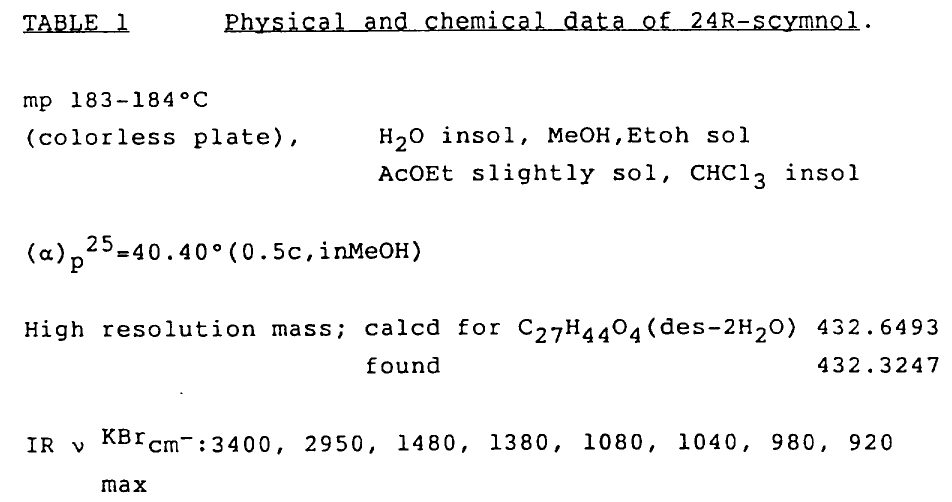

¹H-NMR(in CD₃OD);δ(ppm): 3.95(1H,br), 3.80-3.50(6H,m), 3.48(1H,m), 2.40-2.15(2H,m), 2.10-1.10(23H,m), 1.07(3H,d,J=6.01Hz)0.91(3H,s), 0.71(3H,s)
¹³C-NMR(in CD₃OD);δ(ppm)74.8(d), 73.5(d), 73.0(d), 69.8(d), 62.7(t), 62.0(t), 50.0(t), 49.0(d), 48.1(s), 43.8(d), 43.6(d), 41.7(d), 41.1(t), 37.8(d), 37.2(t), 36.6(s), 36.5(t), 34.0(t), 33.0(t), 31.9(t), 30.3(t), 29.5(t), 28.5(d), 25.0(t), 24.0(q), 18.9(q), 13.8(q).

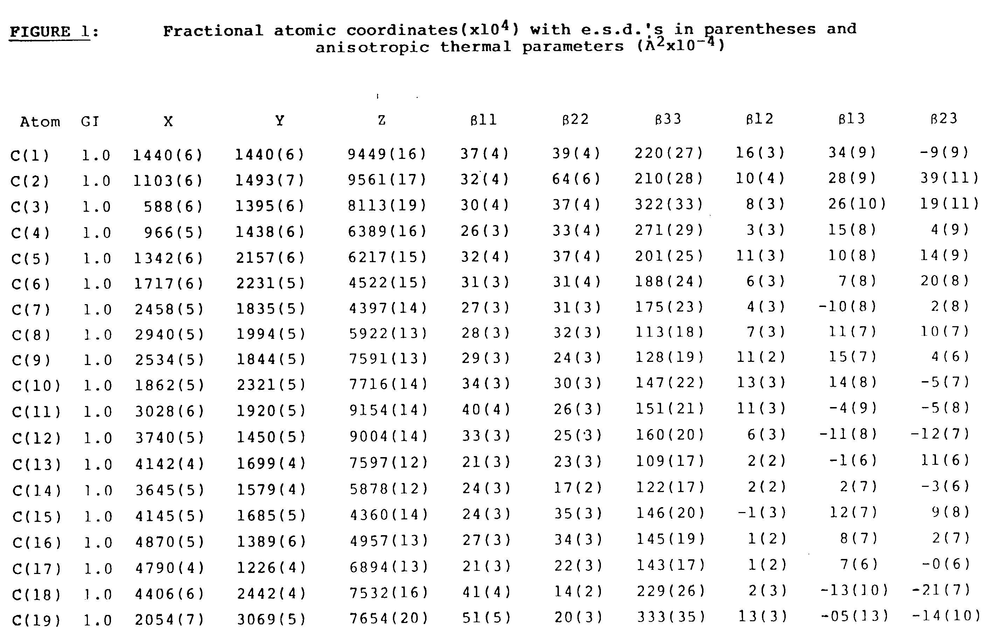

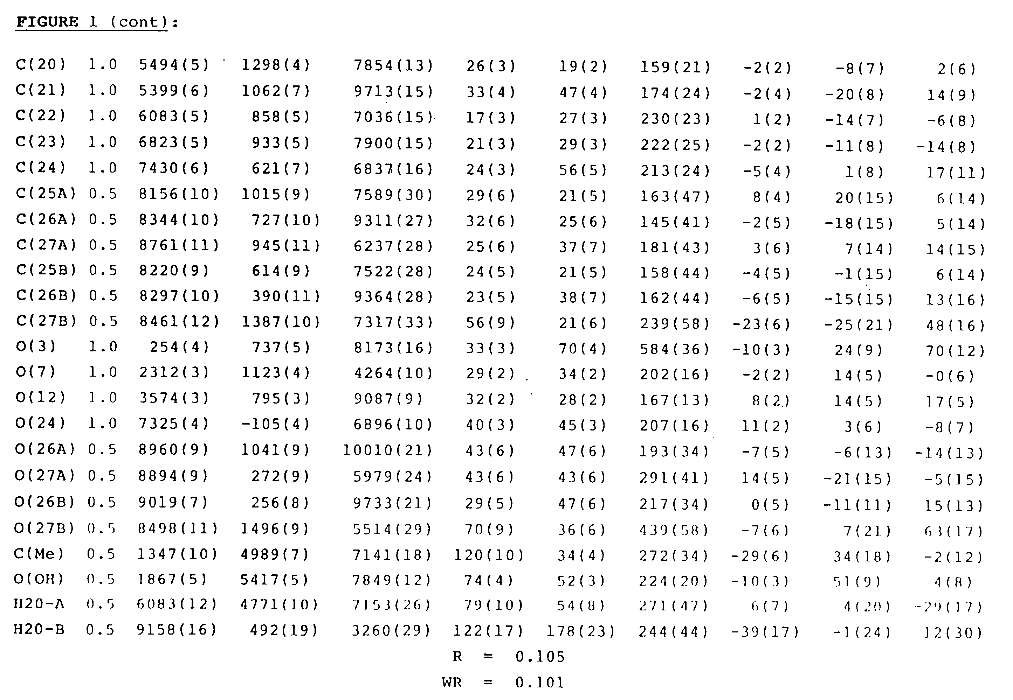

The stereochemical configuration at the 24-position of the compound was determined as R(+) by its crystallographical analysis and the specific optical rotation. The crystal data and atomic parameters of the compound are set out in Table 2 and Figure 1, respectively.

**TABLE 2**

| Crystal data of 24R-scymnol |
|---|
| C₂₇H₄₈O₆.CH₃OH.H₂O, M=518.71, Orthorhombic, Space group P 2₁2₁2, a=18.571(1), b=19.927(2), c=7.984(1)Å, V=2954.8Å³, Z=4, F(000)=1144, Dc=1.19 g/cm³, Do=1.22 g/cm³, λ(Cu=Kα)=1.54180 Å, µ(Cu-Kα)=7.9 cm⁻¹, crystal size 0.2x0.2x0.4 mm. |

The activity of 24R-scymnol in the treatment of liver dysfunction has been investigated. In prior International Patent Application No. PCT/AU87/00281, two assays have been designated to identify characteristic pharmacological activities of the substance, isolutrol. The bioassays, designated as (A) and (B), have been based on the following activities:
(A) the active principle prevented liver damage in mice caused by carbon tetrachloride; and
(B) the active principle increased the respiration rate in mice when a toxic substance, such as nicotine, was administered.

These assays are useful in ascertaining the existence of activity, however it has been found that they are sometimes unreliable and not reproducible, indicating they are not suitable assays for measuring the degree of activity. 24R-scymnol has some activity in these two bioassays, but it is difficult to make comparison of the degree of activity between isolutrol and 24R-scymnol. Accordingly, a new assay has been designed to reproducibly measure the degree of activity. This bioassay, designated as bioassay (C), measures tyrosine aminotransferase (TA) activity in liver of mice. TA is one of important enzymes in liver.

### ASSAY (C):

Mice (5 weeks old) were orally administered the active principle of shark's bile (MD) (10mg/kg), 24R-scymnol (10mg/kg), or water (6:00 PM on the previous day, 9:00 AM on the day). After one hour from the last administration, the mice were forced to swim in a water at 35°C. After 4 hours swimming, mice were sacrificed by decapitation and livers were perfused with 0.145 M KCl to remove blood. Half gram of liver was homogenized in 0.145 M KCl and centrifuged at 10,000xg for 30min. and TA activity in the supernatant was measured by the method of Diammondstone. The activity is shown as the amount of p-hydroxyphenyl pyruvic acid (p-HPP) produced by the enzyme reaction for 10min.

The results of the comparison of the activities of the active principle and 24R-scymnol by the assay (C) are as follows:

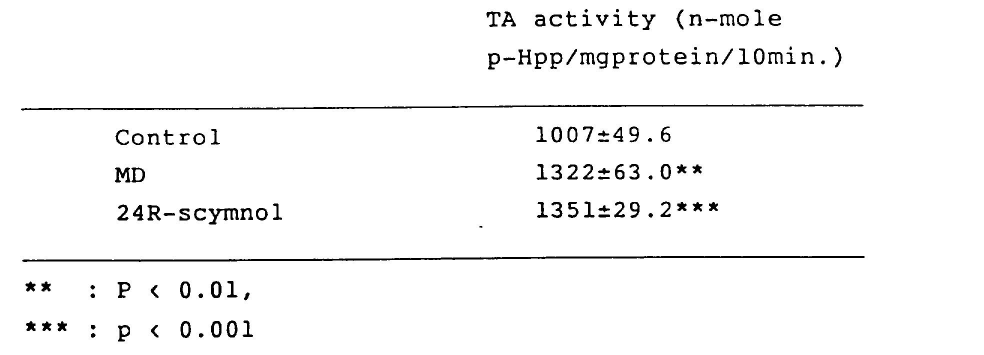

The above results indicate that 24R-scymnol has almost the same activity as the active principle of the shark's bile.

The present invention also provides a pharmaceutical composition comprising 24R-scymnol, together with a pharmaceutically acceptable carrier or diluent therefor. By way of example, 24R-scymnol can be formulated as stable tablets after being mixed as a powder with a known carrier or bulking agent. Such pharmaceutical compositions may be used, for example, for the activation of liver function in the treatment of the diseases of the liver such as hepatitis, nephritis, diabetes, etc.

Further details of this invention will be apparent from the following Examples which illustrate the invention without limiting it in any way.

### EXAMPLE 1 - Preparation of 24R-scymnol.

The active principle of shark's bile (500mg) was dissolved in 7ml of 1% HCl containing 400mg of Bacl₂ and the mixture was heated for 5h at 100°C. After cooling, the resulting solution was extracted three times with 50ml of AcOH-BuOH (1:1). The organic layer was washed twice with H₂O. Removal of the solvent gave a yellow oil. The resultant residue was dissolved in MeOH and applied to reversed phase HPLC. 50mg of 24R-scymnol was obtained.

## Claims

1. 24R-scymnol, having the general formula I:

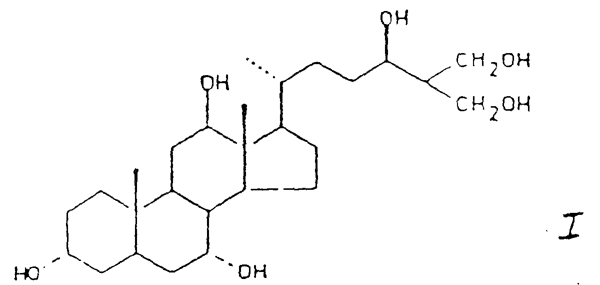

2. The compound of claim 1, in substantially pure form.

3. A method for the preparation of the compound of the general formula I as defined in claim 1, which comprises the step of hydrolysis of a 24R-scymnol sulphate ester with an inorganic acid.

4. A method according to claim 3, wherein said hydrolysis is carried out in the presence of barium chloride.

5. A method according to claim 3 or claim 4, wherein said hydrolysis is carried out with hydrochloric acid.

6. A method according to any one of claims 3 to 5 wherein said 24R-scymnol sulphate ester is sodium 24R-scymnol sulphate.

7. A pharmaceutical composition comprising 24R-scymnol, together with a pharmaceutically acceptable carrier or diluent therefor.

8. 24R-scymnol for use as a medicament

9. The use of 24R-scymnol in the manufacture of a medicament for the treatment of diseases of the liver.

## Patentansprüche

1. 24R-Scymnol mit der allgemeinen Formel I:

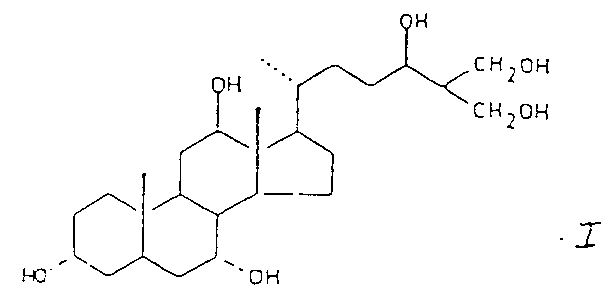

2. Verbindung nach Anspruch 1 in einer im wesentlichen reinen Gestalt.

3. Verfahren zur Herstellung der in Anspruch 1 definierten Verbindung der allgemeinen Formel I, die den Schritt der Hydrolyse eines 24R-Scymnolsulfatesters mit einer anorganischen Säure umfasst.

4. Verfahren nach Anspruch 3, in dem die Hydrolyse in der Gegenwart von Bariumchlorid durchgeführt wird.

5. Verfahren nach Anspruch 3 oder Anspruch 4, in dem die Hydrolyse mit Salzsäure durchgeführt wird.

6. Verfahren nach einem der Ansprüche 3 bis 5, in dem das 24R-Scymnolsulfatester Natrium-24R-Scymnolsulfat ist.

7. Pharmazeutische Zusammensetzung, die 24R-Scymnol zusammen mit einem pharmazeutisch akzeptierbaren Träger oder Verdünnungsmittel dafür umfasst.

8. 24R-Scymnol zur Benutzung als Medikament.

9. Benutzung von 24R-Scymnol bei der Herstellung eines Medikaments zur Behandlung von Erkrankungen der Leber.

## Revendications

1. Scymnol 24R ayant la formule générale I:

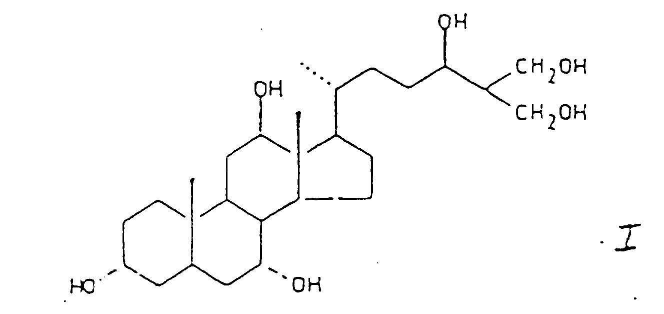

2. Le composé selon la revendication 1 sous forme essentiellement pure.

3. Méthode de préparation du composé de formule générale I tel que défini à la revendication 1, comportant la phase d'hydrolyse d'un ester de sulphate de scymnol 24R avec un acide inorganique.

4. Méthode selon la revendication 3, dont l'hydrolyse est effectuée en présence de chlorure de baryum.

5. Méthode selon la revendication 3 ou la revendication 4, dont l'hydrolyse est effectuée avec l'acide chlorhydrique.

6. Méthode selon l'une ou l'autre des revendications 3 à 5, suivant laquelle l'ester de sulphate de scymnol 24R est le sulphate de scymnol 24R de sodium.

7. Composé pharmaceutique comportant le scymnol 24R, avec un porteur ou diluant prévu à son intention.

8. Scymnol 24R pour l'emploi à titre de médicament.

9. L'exploitation du Scymnol 24 dans la fabrication d'un médicament pour le traitement des maladies du foie.
